# EUROPEAN PATENT APPLICATION

(11) **EP 4 431 530 A1**
(43) Date of publication of application: **18.09.2024**
(21) Application number: 22913996.9
(22) Date of filing: 01.12.2022
(51) Int. Cl.: C07K 19/00, C12N 15/62, A61K 38/16, A61K 48/00

(54) **TRANSGENIC EXPRESSION CASSETTE FOR TREATING MUSCULAR DYSTROPHY**

(30) Priority: 29.12.2021 CN 202111642563
(71) Applicant: Shanghai Mygt Biopharmaceutical LLC, Shanghai 201413 (CN)
(72) Inventor: YAN, Mengdi, Shanghai 201108 (CN); WU, Xia, Shanghai 201108 (CN); XIAO, Xiao, Shanghai 201108 (CN); ZHENG, Jing, Shanghai 201108 (CN); DU, Zengmin, Shanghai 201108 (CN); JIANG, Wei, Shanghai 201108 (CN); CHEN, Hui, Shanghai 201108 (CN); WANG, Liqun, Shanghai 201108 (CN); WANG, Hui, Shanghai 201108 (CN)
(74) Representative: Papula Oy
(86) International application number: PCT/CN2022/135805
(87) International publication number: WO 2023/124741

(57) **Abstract**

A recombinant hybrid protein, a nucleic acid molecule encoding the recombinant hybrid protein, a transgenic expression cassette containing the nucleic acid molecule, and a gene delivery system containing the transgenic expression cassettes that can be used for treating muscular dystrophy. The nucleic acid molecule, the transgenic expression cassette and the gene delivery system can stably express a therapeutic protein in muscle tissues, so that a good therapeutic effect on muscular dystrophy is realized.

## Description

### TECHNICAL FIELD

The present disclosure relates to recombinant hybrid proteins, nucleic acid molecules encoding said recombinant hybrid proteins, transgenic expression cassettes comprising said nucleic acid molecules, and gene delivery systems comprising said transgene expression cassettes that can be used for treating muscular dystrophy.

### BACKGROUND

Muscular dystrophy (MD) refers to a kind of widespread degenerative muscle diseases, which mainly manifests as muscular dysfunction in different areas, such as, muscles progressive muscle atrophy and weakness in varying degrees. Among these, the most common and rapidly progressing one is Duchenne muscular dystrophy (DMD). DMD is a severe X-linked recessive progressive neuromuscular disorder. Due to a lack of dystrophin, patients with DMD experience progressive degeneration of skeletal, heart and respiratory muscles. According to statistics, DMD affects approximately one in 3,600 male infants worldwide. DMD patients gradually lose the ability to walk independently between the ages of 12 to 15, and must rely on wheelchairs. Subsequently, they experience difficulties in breathing, respiratory infections and swallowing problems, and ultimately progressing to cardiomyopathy, leading to death. X-linked recessive muscular dystrophies also include Becker muscular dystrophy (BMD), which is characterized by reduced quantity or quality of dystrophin protein in muscle biopsy samples.

Dystrophin gene, approximately 2.5 Mb in size, is the largest known human gene, and is primarily expressed in skeletal and cardiac muscles. The gene is located on the X chromosome at position Xp21, and contains 79 exons. DMD is caused by mutations in the dystrophin gene. The most common mutations in DMD include large deletion mutations of one or more exons (60-70%), duplication mutations (5-10%), single nucleotide variants (including small deletions or insertions, single-base changes and splice site changes). The basic function of dystrophin protein is to stabilize muscle fibers by bridging and anchoring proteins during contraction, by binding to F-actin through N-terminal domain and binding to β-dystroglycan through C-terminal domain. The deficiency of dystrophin protein expression leads to severe muscle atrophy, respiratory and heart failure, attributed to the loss of dystrophin protein function, disrupting the formation of its dystrophin-associated glycoprotein complex (DGC), leading to membrane instability, increased susceptibility to damage, and fiber necrosis (Findlay A R, Wein N, Kaminoh Y, et al., Clinical phenotypes as predictors of the outcome of skipping around DMD exon 45[J]. Ann Neurol, 2015, 77(4): 668-74). Therefore, increasing the expression of dystrophin protein is critical for the treatment of muscular dystrophy.

So far, there are no effective medicaments for the treatment of muscular dystrophy. Glucocorticoid therapy is the main conservative treatment method, but it can only delay disease progression for one or two years. Therefore, there is an urgent need to obtain effective medicaments for the treatment of muscular dystrophy.

At present, adeno-associated virus (AAV) vector-mediated gene delivery plays a significant role in the treatment of rare monogenic diseases. AAV has low pathogenicity and the ability to achieve long-term stable protein expression in various organs and tissues. These characteristics give AAV clear advantages in the field of gene therapy, making it suitable for delivering therapeutic genes. AAV brings great hope to the treatment of muscular dystrophy. However, the size of genes that can be packaged into AAV vectors is limited, with a maximum of 4.7 kb, making it difficult to carry the large Dystrophin protein gene. It is a challenge for researchers to reduce the size of the large dystrophin protein gene and package it into an AAV expression cassette. Therefore, it is desired to obtain truncated dystrophin protein genes that can stably express functional small dystrophin proteins with therapeutic activity.

Dystrophin-associated protein (Utrophin), a homologous protein of Dystrophin, is also ubiquitously expressed in muscle and has similar functions and structures to Dystrophin (Miura P, Jasmin B J. Utrophin upregulation for treating Duchenne or Becker muscular dystrophy: how close are we?[J]. Trends Mol Med, 2006, 12(3): 122-9; Fairclough R J, Wood M J, Davies K E. Therapy for Duchenne muscular dystrophy: renewed optimism from genetic approaches [J]. Nat Rev Genet, 2013, 14(6): 373-8). Studies have shown that up-regulation of Utrophin expression also plays an important role in the treatment of muscular dystrophy and may also be used as a therapeutic target for muscular dystrophy (Guiraud S, Chen H, Burns D T et al., Advances in genetic therapeutic strategies for Duchenne muscular dystrophy[J]. Exp Physiol, 2015, 100(12): 1458-67; Moorwood C, Khurana T S. Duchenne muscular dystrophy medicament discovery - the application of utrophin promoter activation screening[J]. Expert Opin Medicament Discov, 2013, 8(5): 569-81; Ricoti V, Spinty S, Roper H, et al., Safety, Tolerability, and Pharmacokinetics of SMT C1100, a 2-Arylbenzoxazole Utrophin Modulator, following Single- and Multiple-Dose Administration to Pediatric Patients with Duchenne Muscular Dystrophy[J]. PLoS One, 2016, 11(4): e0152840). Furthermore, studies have shown that the up-regulation of the expression of the dystrophin-associated protein Utrophin through small molecule compounds or microRNA can alleviate the symptoms of muscular dystrophy.

### SUMMARY

In order to solve the above-mentioned technical problems, in a first aspect, the present disclosure provides a recombinant hybrid protein, comprising: N-terminal domain, hinge H1, spectrin-like repeat R1, spectrin-like repeat R2, spectrin-like repeat R3 and a first half of hinge H2 of a full-length human dystrophin-associated protein (Utrophin); and, spectrin-like repeat R23, spectrin-like repeat R24, hinge H4 and CR domain of a full-length human Dystrophin.

In one embodiment, the recombinant hybrid protein comprises an amino acid sequence as shown in SEQ ID NO: 4. In a preferred embodiment, the recombinant hybrid protein consists of the amino acid sequence as shown in SEQ ID NO: 4.

The recombinant hybrid protein of the present disclosure can effectively improve muscle function, alleviate the symptoms of muscular dystrophy, and has good therapeutic effects on muscular dystrophy.

In a second aspect, the present disclosure provides a nucleic acid molecule encoding the recombinant hybrid protein according to the first aspect.

In one embodiment, the nucleic acid molecule has a nucleotide sequence at least 50% identical to the nucleotide sequence shown in SEQ ID NO: 8 or SEQ ID NO: 10, preferably at least 50%, 60%, 70%, 80%, 85%, 90%, 95%, 99% or 100% identical to the nucleotide sequence shown in SEQ ID NO: 8 or SEQ ID NO: 10.

In one embodiment, the nucleic acid molecule comprises a nucleotide sequence as shown in SEQ ID NO: 8 or SEQ ID NO: 10. In a preferred embodiment, the nucleic acid molecule consists of the nucleotide sequence as shown in SEQ ID NO: 8 or SEQ ID NO: 10.

In a third aspect, the present disclosure provides a transgenic expression cassette, comprising: a promoter, the nucleic acid molecule according to the second aspect, and a mini polyA.

In one embodiment, the promoter is selected from: CB promoter, CAG promoter, or promoters of muscle-specific genes including muscle creatine kinase (MCK) promoter, human creatine kinase (hCK) promoter, shortened human creatine kinase (shCK) promoter, skeletal muscle α-actin promoter, cardiac α-actin promoter, myosin heavy chain (MyHC) promoter, myosin light chain 2 (MLC2) promoter, myosin light chain 3F promoter, desmin gene promoter, and myogenic regulatory factor family (MyoG, Myf5, Mrf4 and Myogenin). In a preferred embodiment, the promoter is hCK promoter or shCK promoter. In a more preferred embodiment, the promoter is shCK promoter. Higher levels of protein expression can be obtained by using shCK compared to hCK.

In one embodiment, the promoter has a nucleotide sequence as shown in SEQ ID NO: 9.

In one embodiment, the transgenic expression cassette further comprises regulatory elements, such as two ITRs located at its both ends, each of which is either a normal ITR or a shortened ITR, such as a normal ITR of 145 bp or a shortened ITR of 100 bp. In a preferred embodiment, the two ITRs are both normal ITRs of 145 bp.

In one embodiment, the transgene expression cassette further comprises an origin of replication, a polyadenylation signal, an internal ribosome entry site (IRES), and/or a 2A signal, such as P2A, T2A, and F2A.

In one embodiment, the transgenic expression cassette consists of a nucleotide sequence as shown in SEQ ID NO: 3, SEQ ID NO: 5, SEQ ID NO: 6 or SEQ ID NO: 7.

In a fourth aspect, the present disclosure provides a gene delivery system, comprising: the transgenic expression cassette according to the third aspect and an AAV capsid protein.

In one embodiment, the AAV capsid protein is a natural AAV capsid protein or an engineered AAV capsid protein. In a preferred embodiment, the AAV is selected from: AAV1, AAV2, AAV3, AAV4, AAV5, AAV6, AAV7, AAV8, AAV9, AAV10, AAV11, AAV12, AAV-DJ, AAV-DJ8, AAV-DJ9, AAVrh8, AAVrh8R, AAVrh10, AAVrh39, AAVrh43, AAV32.33, AAV3B, AAVv66, AAVXL32 and AAVPHP.B.

The nucleic acid molecules, transgenic expression cassettes and gene delivery systems of the present disclosure can stably express the above-mentioned recombinant hybrid protein in muscle tissue, thereby improving muscle function and achieving good therapeutic effects on muscular dystrophy.

In a fifth aspect, the present disclosure provides the use of the transgenic expression cassette according to the third aspect or the gene delivery system according to the fourth aspect in the preparation of a pharmaceutical composition for treating muscular dystrophy.

In one embodiment, the muscular dystrophy comprises Duchenne muscular dystrophy, Becker muscular dystrophy, and other muscle degenerative diseases.

In a preferred embodiment, the muscular dystrophy is Duchenne muscular dystrophy.

In a sixth aspect, the present disclosure provides a pharmaceutical composition, comprising: one of the recombinant hybrid protein according to the first aspect, the nucleic acid molecule according to the second aspect, the transgenic expression cassette according to the third aspect and the gene delivery system according to the fourth aspect; and excipient(s).

In one embodiment, the pharmaceutical composition is used for treating muscular dystrophy, wherein the muscular dystrophy comprises: Duchenne muscular dystrophy, Becker muscular dystrophy, and other muscle degenerative diseases. In a preferred embodiment, the muscular dystrophy is Duchenne muscular dystrophy.

In a seventh aspect, the present disclosure provides a method of treating muscular dystrophy, comprising: administering to a subject in need thereof a therapeutically effective amount of the pharmaceutical composition according to the sixth aspect.

In one embodiment, the pharmaceutical composition is administered through systemic or local routes, such as by intravenous administration, intramuscular administration, subcutaneous administration, oral administration, local administration, intraperitoneal administration or intralesional administration.

In a preferred embodiment, the pharmaceutical composition is administered through systemic route, for example, by intravenous administration.

In a preferred embodiment, the pharmaceutical composition is administered to muscle through local route, for example, the pharmaceutical composition is injected into biceps brachii or gastrocnemius muscle.

### BRIEF DESCRIPTION OF THE DRAWINGS

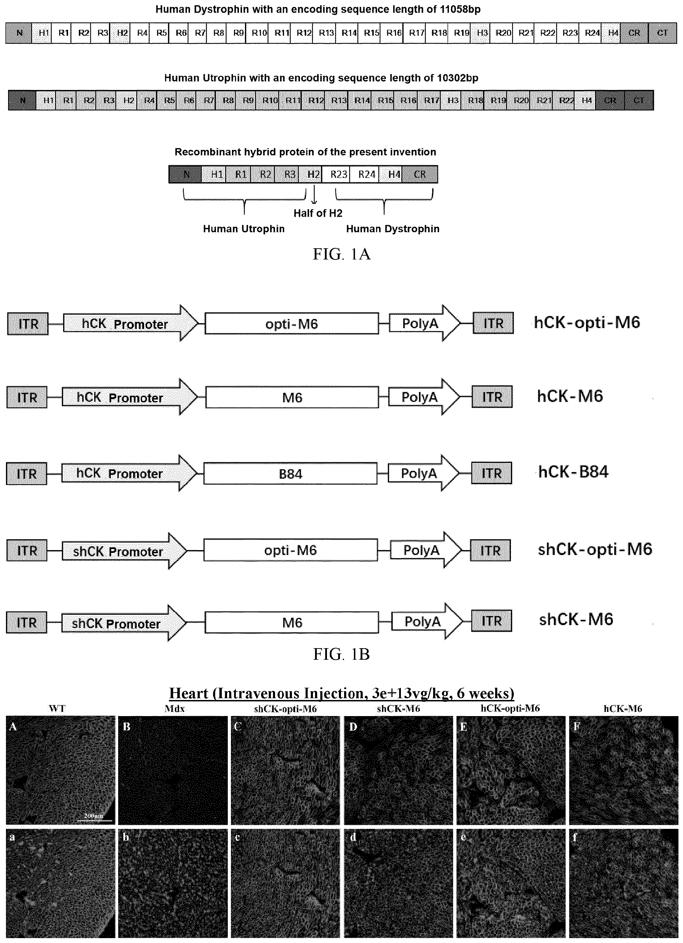

FIG. 1A shows the structures (subdomains) of the full-length human dystrophin protein, the full-length human dystrophin-associated protein, and the recombinant hybrid protein encoded by the M6 construct constructed by the present inventors.
FIG. 1B shows schematic diagrams of hCK-opti-M6, hCK-M6, hCK-B84, shCK-opti-M6 and shCK-M6 expression cassettes according to one embodiment.

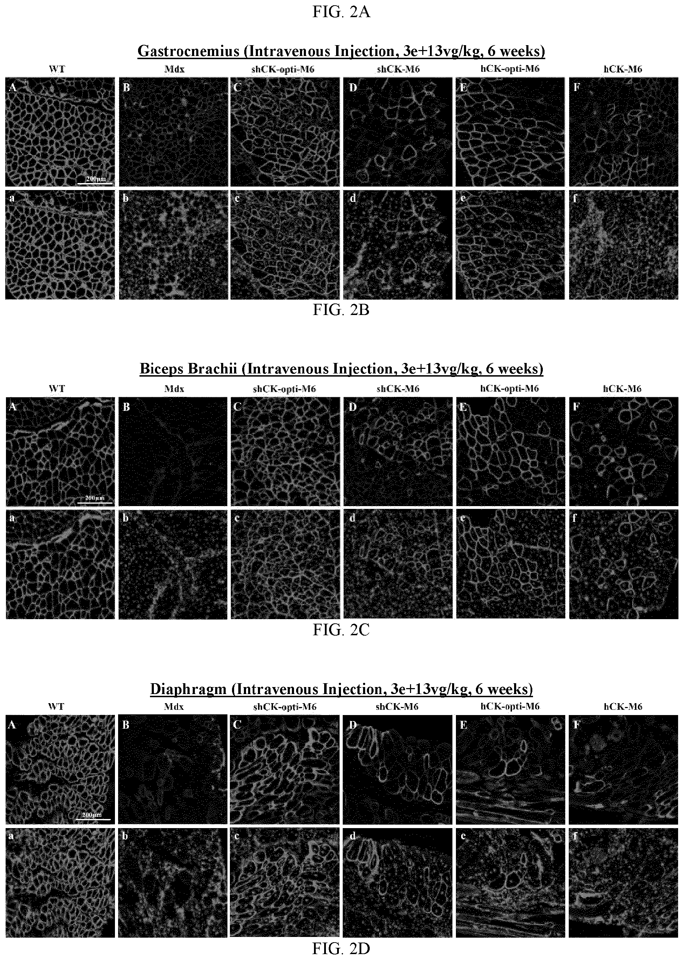

FIG. 2A shows the immunofluorescence analysis of therapeutic proteins in the hearts of mice injected with AAV9-hCK-opti-M6, AAV9-hCK-M6, AAV9-shCK-opti-M6, and AAV9-shCK-M6 six weeks post-injection. Frozen sections of the hearts from WT mice, Mdx mice and mice in the treatment groups were immunofluorescently stained with antibodies, and cell nuclei were counterstained with DAPI. Scale bar: 200µm.
FIG. 2B shows the immunofluorescence analysis of therapeutic proteins in the gastrocnemius muscles of mice injected with AAV9-hCK-opti-M6, AAV9-hCK-M6, AAV9-shCK-opti-M6, and AAV9-shCK-M6 six weeks post-inj ection. Frozen sections of the gastrocnemius muscles from WT mice, Mdx mice and mice in the treatment groups were immunofluorescently stained with antibodies, and cell nuclei were counterstained with DAPI. Scale bar: 200µm.
FIG. 2C shows the immunofluorescence analysis of therapeutic proteins in the biceps brachii muscles of mice injected with AAV9-hCK-opti-M6, AAV9-hCK-M6, AAV9-shCK-opti-M6, and AAV9-shCK-M6 six weeks post-injection. Frozen sections of biceps brachii muscles from WT mice, Mdx mice and mice in the treatment groups were immunofluorescently stained with antibodies, and cell nuclei were counterstained with DAPI. Scale bar: 200µm.
FIG. 2D shows the immunofluorescence analysis of therapeutic proteins in the diaphragms of mice injected with AAV9-hCK-opti-M6, AAV9-hCK-M6, AAV9-shCK-opti-M6, and AAV9-shCK-M6 six weeks post-injection. Frozen sections of the diaphragms from WT mice, Mdx mice and mice in the treatment groups were immunofluorescently stained with antibodies, and cell nuclei were counterstained with DAPI. Scale bar: 200µm.

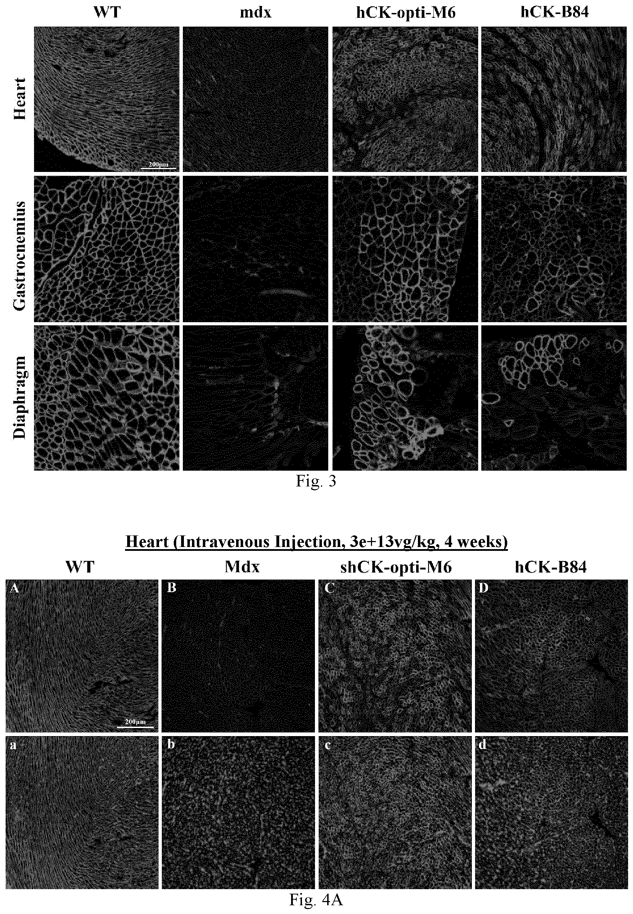

FIG. 3 shows a comparison of the expressions of the opti-M6 sequence and the existing B84 sequence in mice. Frozen sections of the hearts, gastrocnemius muscles, and diaphragms from WT mice, Mdx mice, mice in the treatment groups of hCK-opti-M6 and hCK-B84 were immunofluorescently stained with antibodies, and cell nuclei were counterstained with DAPI. Scale bar: 200µm.

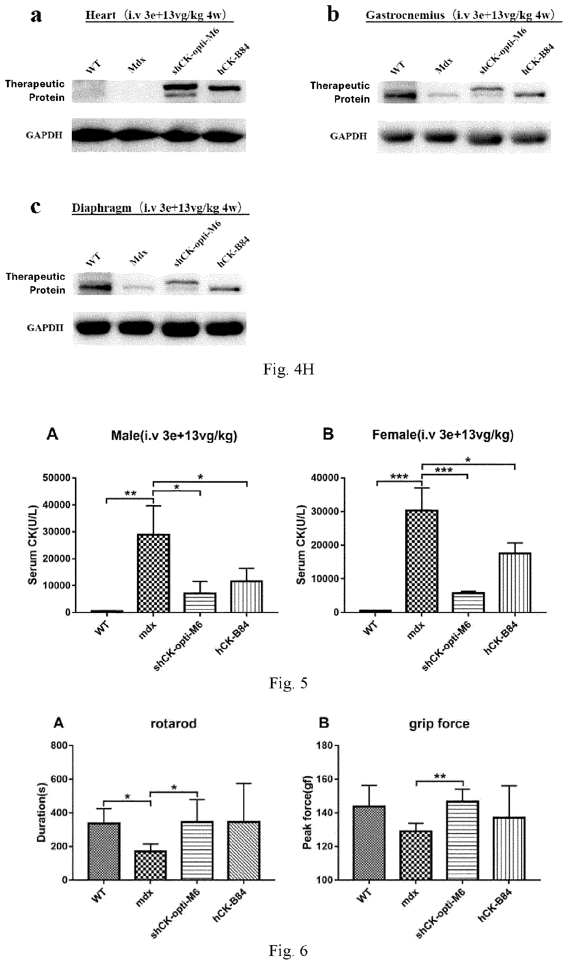

FIG. 4A shows the immunofluorescence analysis of therapeutic proteins in the hearts of mice injected with AAV9-shCK-opti-M6 and AAV9-hCK-B84 four weeks post-injection. Frozen sections of the hearts from WT mice, Mdx mice and mice in the two treatment groups were immunofluorescently stained with antibodies, and cell nuclei were counterstained with DAPI. Scale bar: 200 µm.

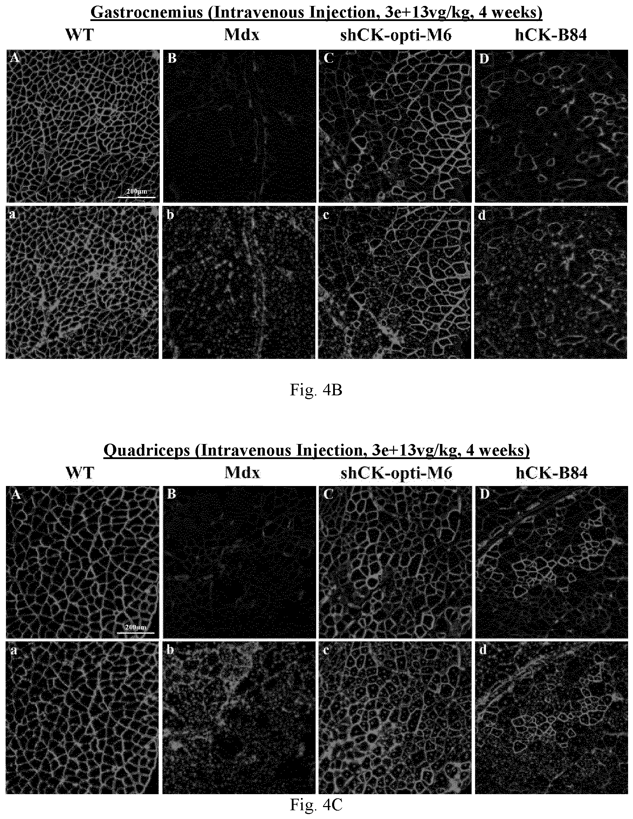

FIG. 4B shows the immunofluorescence analysis of therapeutic proteins in the gastrocnemius muscle of mice injected with AAV9-shCK-opti-M6 and AAV9-hCK-B84 four weeks post-injection. Frozen sections of gastrocnemius muscles form WT mice, Mdx mice and mice in the two treatment groups were immunofluorescently stained with antibodies, and cell nuclei were counterstained with DAPI. Scale bar: 200 µm.
FIG. 4C shows the immunofluorescence analysis of therapeutic proteins in the quadriceps muscle of mice injected with AAV9-shCK-opti-M6 and AAV9-hCK-B84 four weeks post-injection. Frozen sections of quadriceps muscles from WT mice, Mdx mice and mice in the two treatment groups were immunofluorescently stained with antibodies, and cell nuclei were counterstained with DAPI. Scale bar: 200 µm.

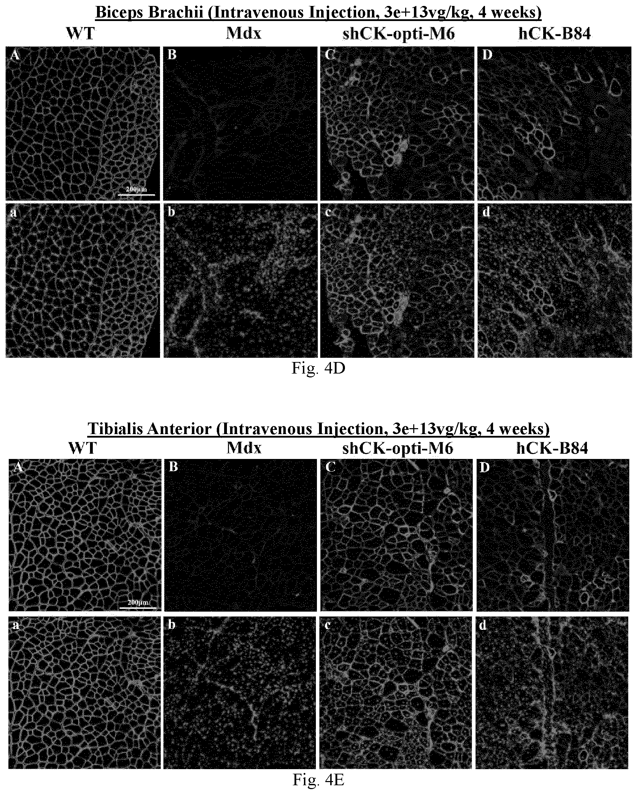

FIG. 4D shows the immunofluorescence analysis of therapeutic proteins in the biceps brachii muscles of mice injected with AAV9-shCK-opti-M6 and AAV9-hCK-B84 four weeks post-injection. Frozen sections of biceps brachii muscles from WT mice, Mdx mice and mice in two treatment groups were immunofluorescently stained with antibodies, and cell nuclei were counterstained with DAPI. Scale bar: 200 µm.
FIG. 4E shows the immunofluorescence analysis of therapeutic proteins in the tibialis anterior muscles of mice injected with AAV9-shCK-opti-M6 and AAV9-hCK-B84 four weeks post-injection. Frozen sections of the tibialis anterior muscles from WT mice, Mdx mice and mice in the two treatment groups were immunofluorescently stained with antibodies, and cell nuclei were counterstained with DAPI. Scale bar: 200µm.

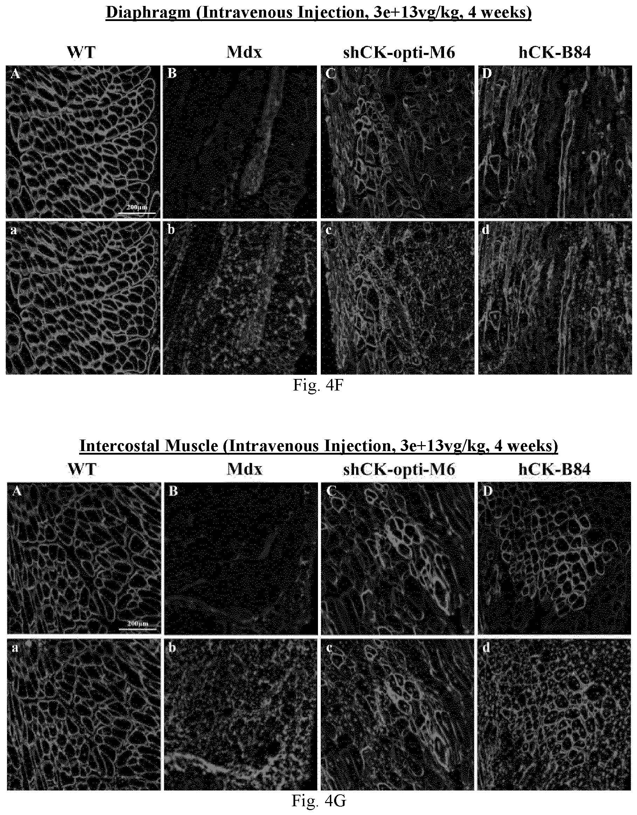

FIG. 4F shows the immunofluorescence analysis of therapeutic proteins in the diaphragms of mice injected with AAV9-shCK-opti-M6 and AAV9-hCK-B84 four weeks post-injection. Frozen sections of the diaphragms from WT mice, Mdx mice and mice in the two treatment groups were immunofluorescently stained with antibodies, and cell nuclei were counterstained with DAPI. Scale bar: 200 µm.
FIG. 4G shows the immunofluorescence analysis of therapeutic proteins in the intercostal muscles of mice injected with AAV9-shCK-opti-M6 and AAV9-hCK-B84 four weeks post-injection. Frozen sections of the intercostal muscles from WT mice, Mdx mice and mice in the two treatment groups were immunofluorescently stained with antibodies, and cell nuclei were counterstained with DAPI. Scale bar: 200 µm.
FIG. 4H shows the expression levels of the shCK-opti-M6 expression cassette and the hCK-B84 expression cassette. Western blot analysis of (a) hearts, (b) gastrocnemius muscles, and (c) diaphragms from WT mice, Mdx mice, and mice treated with AAV9-shCK-opti-M6 and AAV9-hCK-B84 for four weeks. GAPDH was used as an internal reference.
FIG. 5 shows the serum creatine kinase (CK) in male and female mice. (A) depicts the serum CK levels of male WT mice, Mdx mice, and mice treated with AAV9-shCK-opti-M6 and AAV9-hCK-B84 for 8-9 weeks. (B) depicts the serum CK level of female WT mice, Mdx mice, and mice treated with AAV9-shCK-opti-M6 and AAV9-hCK-B84 for 8-9 weeks. n=4, *p<0.05, **p<0.01, ***p<0.001, t-test.
FIG. 6 shows the improvements in muscle function and behavior in mice. (A) Rotarod performance test and (B) grip force test of WT mice, Mdx mice, and mice treated with AAV9-shCK-opti-M6 and AAV9-hCK-B84 for 8 weeks. n=4, *p<0.05, **p<0.01, t-test.

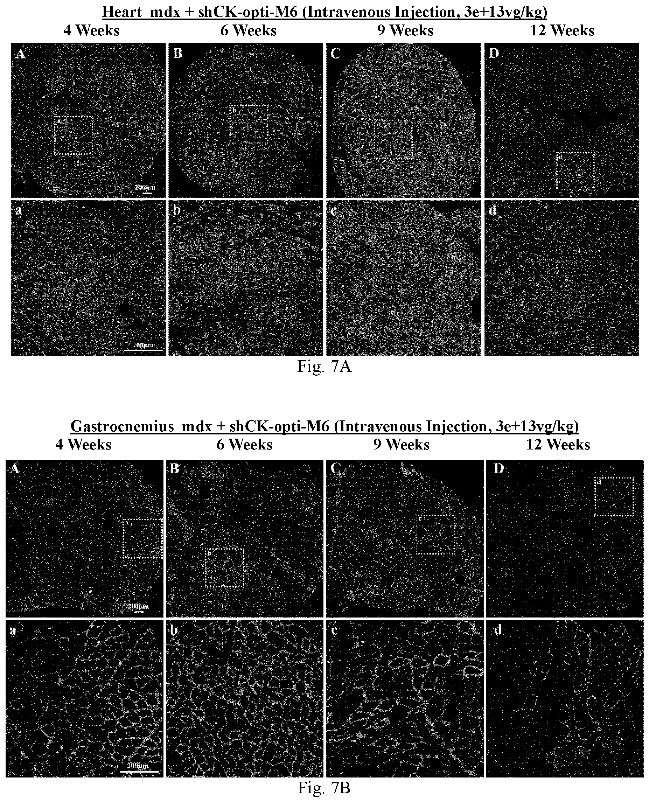

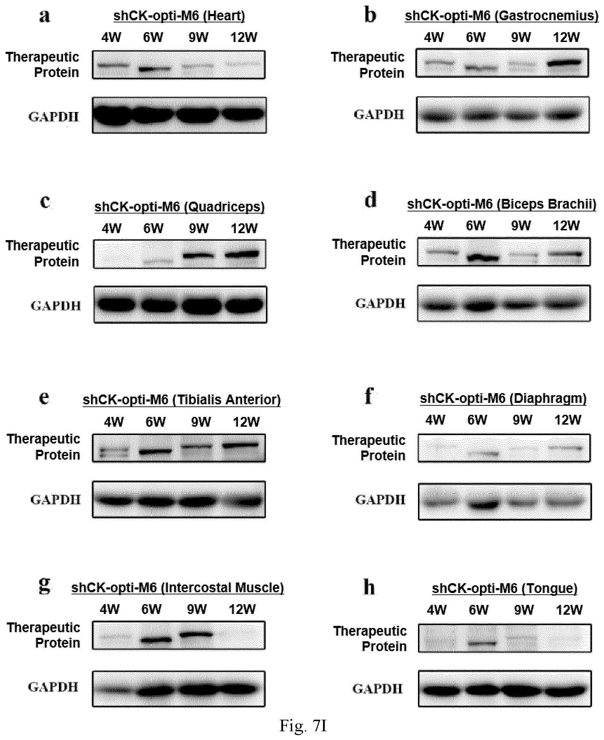

FIG. 7A shows the long-term expression of AAV9-shCK-opti-M6 in the hearts of mice. Immunofluorescence analysis of therapeutic proteins was conducted in the hearts of mice at different time points post-injection (4 weeks, 6 weeks, 9 weeks and 12 weeks post-injection). Immunofluorescence staining was performed with antibodies, and cell nuclei were counterstained with DAPI. Scale bar: 200µm.
FIG. 7B shows the long-term expression of AAV9-shCK-opti-M6 in the gastrocnemius muscles of mice. Immunofluorescence analysis of therapeutic proteins was conducted in the gastrocnemius muscles of mice at different time points post-injection (4 weeks, 6 weeks, 9 weeks and 12 weeks post-injection). Immunofluorescence staining was performed with antibodies, and cell nuclei were counterstained with DAPI. Scale bar: 200µm.

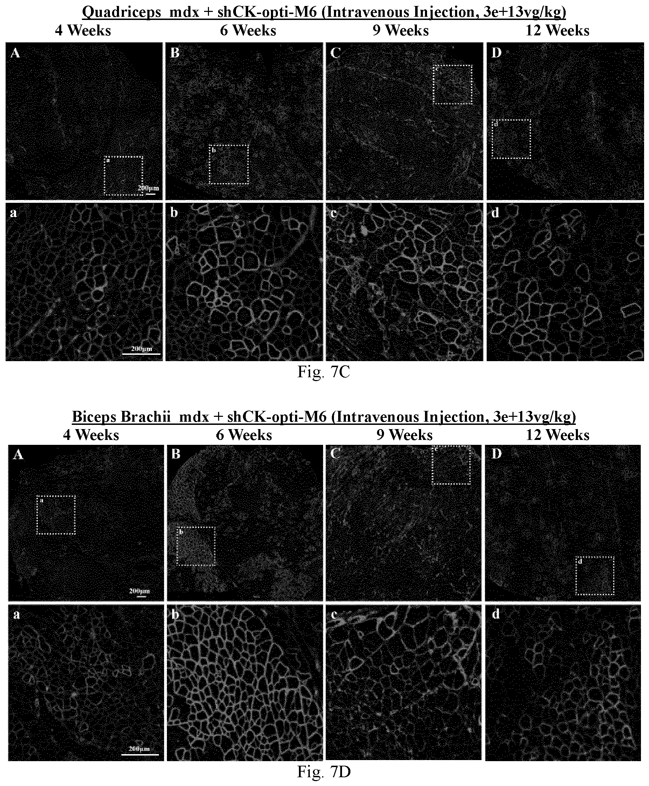

FIG. 7C shows the long-term expression of AAV9-shCK-opti-M6 in the quadriceps muscles of mice. Immunofluorescence analysis of therapeutic proteins was conducted in the quadriceps muscles of mice at different time points post-injection (4 weeks, 6 weeks, 9 weeks and 12 weeks post-injection). Immunofluorescence staining was performed with antibodies, and cell nuclei were counterstained with DAPI. Scale bar: 200µm.
FIG. 7D shows the long-term expression of AAV9-shCK-opti-M6 in the biceps brachii muscles of mice. Immunofluorescence analysis of therapeutic proteins was conducted in the biceps brachii muscles of mice at different time points post-injection (4 weeks, 6 weeks, 9 weeks and 12 weeks post-injection). Immunofluorescence staining was performed with antibodies, and cell nuclei were counterstained with DAPI. Scale bar: 200µm.

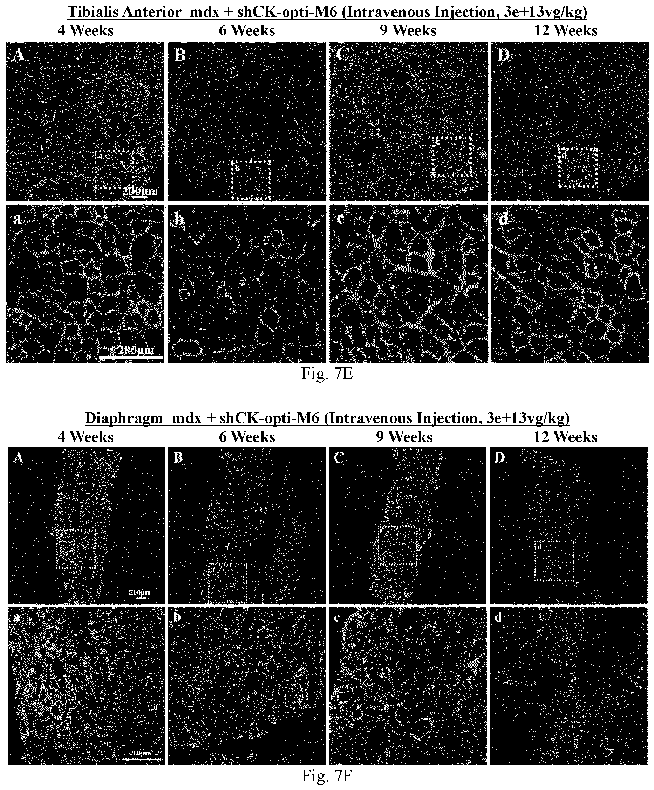

FIG. 7E shows the long-term expression of AAV9-shCK-opti-M6 in the tibialis anterior muscles of mice. Immunofluorescence analysis of therapeutic proteins was conducted in the tibialis anterior muscles of mice at different time points post-injection (4 weeks, 6 weeks, 9 weeks and 12 weeks post-injection). Immunofluorescence staining was performed with antibodies, and cell nuclei were counterstained with DAPI. Scale bar: 200µm.
FIG. 7F shows the long-term expression of AAV9-shCK-opti-M6 in the diaphragms of mice. Immunofluorescence analysis of therapeutic proteins was conducted in the diaphragms of mice at different time points post-injection (4 weeks, 6 weeks, 9 weeks and 12 weeks post-injection). Immunofluorescence staining was performed with antibodies, and cell nuclei were counterstained with DAPI. Scale bar: 200µm.

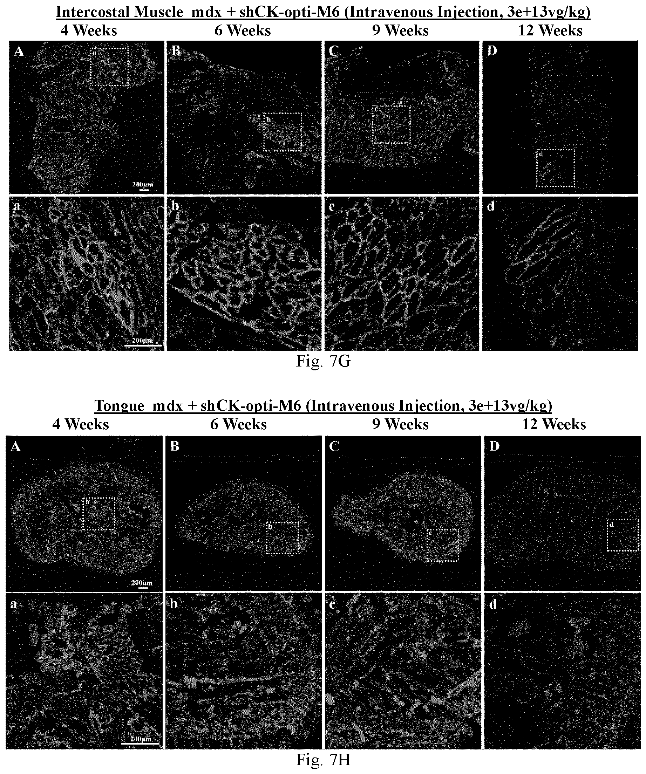

FIG. 7G shows the long-term expression of AAV9-shCK-opti-M6 in the intercostal muscles of mice. Immunofluorescence analysis of therapeutic proteins was conducted in the intercostal muscles of mice at different time points post-injection (4 weeks, 6 weeks, 9 weeks and 12 weeks post-injection). Immunofluorescence staining was performed with antibodies, and cell nuclei were counterstained with DAPI. Scale bar: 200µm.
FIG. 7H shows the long-term expression of AAV9-shCK-opti-M6 in the tongues of mice. Immunofluorescence analysis of therapeutic proteins was conducted in the tongues of mice at different time points post-injection (4 weeks, 6 weeks, 9 weeks and 12 weeks post-injection). Immunofluorescence staining was performed with antibodies, and cell nuclei were counterstained with DAPI. Scale bar: 200µm.
FIG. 7I shows the long-term expression levels of shCK-opti-M6 expression cassette in mice *in vivo.* West blot analysis of the (a) hearts, (b) gastrocnemius muscles, (c) quadriceps muscles, (d) biceps brachii muscles, (e) tibialis anterior muscles, (f) diaphragms, (g) intercostal muscles, and (h) tongues from the mice treated with AAV9-shCK-opti-M6 for 4 weeks, 6 weeks, 9 weeks and 12 weeks. GAPDH was used as an internal reference.

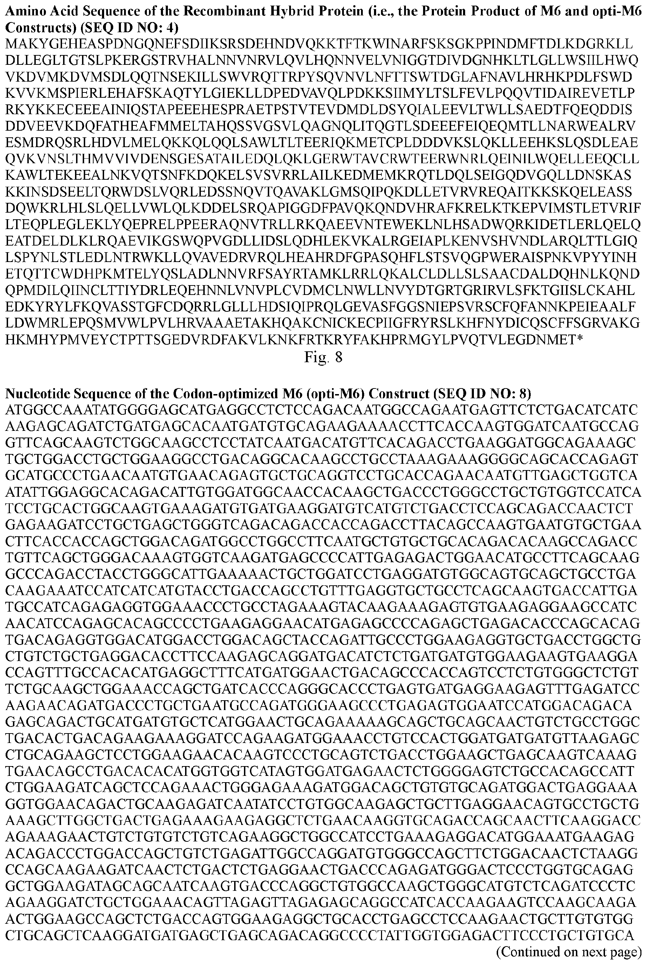

FIG. 8 shows the amino acid sequence of the recombinant hybrid protein (i.e., the protein product of M6 and opti-M6 constructs) (SEQ ID NO: 4).

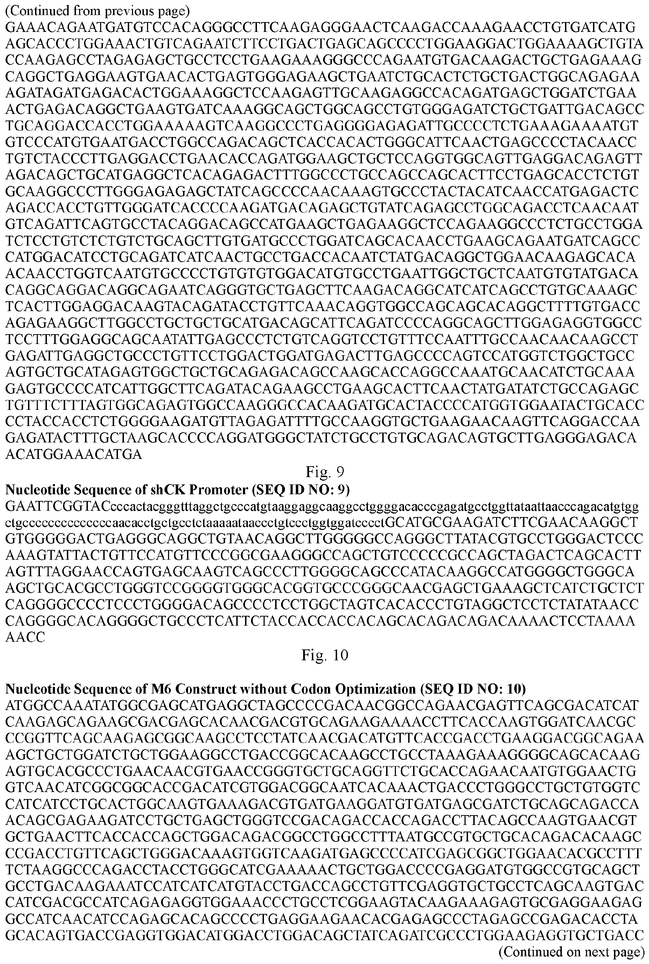

FIG. 9 shows the nucleotide sequence of the codon-optimized M6 (opti-M6) construct (SEQ ID NO: 8).
FIG. 10 shows the nucleotide sequence of the shCK promoter (SEQ ID NO: 9).

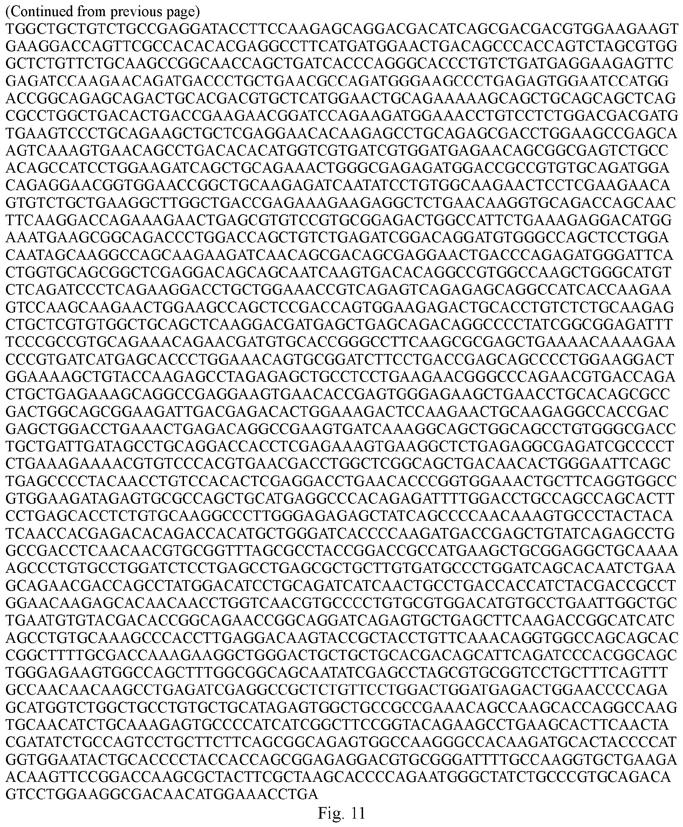

FIG. 11 shows the nucleotide sequence of the M6 construct without codon optimization (SEQ ID NO: 10).

### DETAILED DESCRIPTION

Unless otherwise defined, all technical and scientific terms used herein have the same meaning as commonly understood by one of ordinary skill in the art to which this disclosure belongs.

Unless otherwise indicated, nucleic acid or polynucleotide sequences are presented herein in the form of single strand, in the 5' to 3' direction, from left to right. Nucleotide and amino acids presented herein are in the format recommended by the IUPACIUB Biochemical Nomenclature Commission, or (for amino acids) by either the one-letter code or the three-letter code.

Unless otherwise indicated, "polynucleotide" is a synonym for "nucleic acid" and refers to a polymeric form of nucleotides of any length, including deoxyribonucleotides or ribonucleotides, their hybrid sequences or analogs. A polynucleotide may include modified nucleotides, such as methylated or capped nucleotides and nucleotide analogs.

As used herein, the terms such as "comprising", "having", "including", and "containing" shall be construed as open-ended phrases (i.e., meaning "including but not limited to").

As used herein, the terms "patient" and "subject" are used interchangeably and in their conventional sense to refer to an organism that suffers from or is susceptible to a condition that can be prevented or treated by administration of the pharmaceutical composition of the present disclosure, and include humans and non-human animals (e.g., rodents or other mammals).

In one embodiment, the subject is a non-human animal (e.g., chimpanzees and other ape and monkey species; farm animals, such as cattle, sheep, pigs, goats, and horses; domestic mammals, such as dogs and cats; experimental animals, including rodents, such as mice, rats and guinea pigs; birds, including domestic, wild and game birds, such as chickens, turkeys and other gallinaceous birds, ducks, geese, etc.). In one embodiment, the subject is a mammal. In one embodiment, the subject is a human.

As used herein, the terms "treat", "treating", and "treatment" (and grammatical variations thereof) includes: (1) inhibiting a condition, disease or disorder, that is, preventing, reducing or delaying the progression of the disease or its recurrence or the development of at least one clinical or subclinical symptom thereof; or (2) alleviating a disease, that is, causing resolution of a condition, disease or condition or at least one of its clinical or subclinical symptoms.

As used herein, the term "therapeutically effective amount" refers to a dose that produces the therapeutic effect for which it is administered. For example, a therapeutically effective amount of a medicament suitable for treating muscular dystrophy may be an amount sufficient to prevent or ameliorate one or more symptoms associated with the muscular dystrophy.

As used herein, the terms "improve", "improving", and "improvement" (and grammatical variations thereof) refers to an improvement in a symptom associated with a disease, and may refer to an improvement in at least one parameter that measures or quantifies the symptom.

As used herein, the terms "prevent", "preventing", and "prevention" (and grammatical variations thereof) a condition, disease or disorder includes: preventing, delaying or reducing the incidence and/or likelihood of the occurrence of at least one clinical or subclinical symptom of the condition, disease or disorder developing in a subject who may have or be susceptible to the condition, disease, or disorder but has not yet experienced or exhibited clinical or subclinical symptoms of the condition, disease, or disorder.

As used herein, the term "local administration" or "local route" refers to administration with local effects.

As used herein, the terms "transduce", "transducing", "transduction", "transfect", "transfecting", "transfection", "transform", "transforming", and "transformation" (and grammatical variations thereof) refer to a process of delivering an exogenous nucleic acid into a host cell, followed by the transcription and translation of the polynucleotide product. Such process includes the use of recombinant viruses to introduce exogenous polynucleotides into host cells.

As used herein, the term "gene delivery" (and grammatical variations thereof) refers to the introduction of an exogenous polynucleotide into a cell for gene transfer, and includes targeting, binding, uptake, transport, and replicon integration and expression.

As used herein, the terms "gene expression" or "express", "expressing", and "expression" (and grammatical variations thereof) refer to the process of gene transcription, translation, and post-translational modification to produce the RNA or protein products of the gene.

As used herein, the terms "infect", "infecting", and "infection" (and grammatical variations thereof) refer to the process by which a virus or viral particle containing a polynucleotide component delivers the polynucleotide into a cell and produces its RNA and protein products. It may also refer to the process of viral replication in a host cell.

As used herein, the term "targeting" (and grammatical variations thereof) means that the virus preferentially enters some cells or tissues and then further expresses the sequence carried by the viral genome or recombinant transgene in the cells.

As used herein, the term "vector" refers to one or series of macromolecules that encapsulates a polynucleotide, which facilitate the delivery of the polynucleotide to a target cell either *in vitro* or *in vivo.* Categories of vectors include, but not limited to, plasmids, viral vectors, liposomes, and other gene delivery vectors. The polynucleotide to be delivered, sometimes referred to as an "expression cassette" or "transgenic cassette," may include, but not limited to, an encoding sequence of certain proteins or synthetic polypeptides that may enhance, inhibit, attenuate, protect, trigger, or prevent certain biological and physiological functions, an encoding sequence of interest in vaccine development (e.g., a polynucleotide expressing a protein, polypeptide, or peptide suitable for eliciting an immune response in mammals), an encoding sequence of RNAi materials (e.g., shRNA, siRNA, antisense oligonucleotides), or an optional biomarker.

As used herein, the terms "expression cassette", "transgenic cassette" and "transgenic expression cassette" are used interchangeably, and refer to a polynucleotide fragment encoding a specific protein, polypeptide or RNAi element that can be cloned into a plasmid vector.

In some embodiments, a "cassette" can also be packaged into AAV particles and used as a viral genome to deliver transgenic products into target cells. The "cassette" may also include other regulatory elements, such as specific promoters/enhancers, polyA, regulatory introns, etc., to enhance or reduce the expression of the transgenic product.

In one embodiment, in addition to the sequence encoding the protein product, the transgenic cassette further contains a number of regulatory elements to enable packaging of the transgene into the virus, such as a normal ITR of 145 bp, a shortened ITR of approximately 100 bp in length. In some embodiments, the transgenic cassette further contains polynucleotide elements for controlling the expression of protein products, such as an origin of replication, a polyadenylation signal, an internal ribosome entry site (IRES), or a 2A signal (e.g., P2A, T2A, F2A), promoters and enhancers, e.g., CMV promoter or other hybrid CMV promoters (called as CB and CAG promoters) with vertebrate β-actin, β-globin or β-globin regulatory elements, or promoters of muscle-specific genes including muscle creatine kinase (MCK) promoter, human creatine kinase (hCK) promoter, shortened creatine kinase (shCK) promoter, skeletal muscle α-actin promoter, cardiac α-actin promoter, myosin heavy chain (MyHC) promoter, myosin light chain 2 (MLC2) promoter, myosin light chain 3F promoter, desmin gene promoter, and myogenic regulatory factor family (MyoG, Myf5, Mrf4 and Myogenin). Promoters and enhancers can be activated by chemicals or hormones (such as doxycycline or tamoxifen) to ensure the gene expression at specific time points. Furthermore, promoters and enhancers can be natural or artificial or chimeric sequences, that is, prokaryotic or eukaryotic sequences.

In some preferred embodiments, inducible regulatory elements for gene expression can be tissue- or organ-specific promoters or enhancers, including but not limited to: promoters specific for various types of muscle cells, e.g., promoters of muscle-specific genes including muscle creatine kinase (MCK) promoter, human creatine kinase (hCK) promoter, shortened human creatine kinase (shCK) promoter, skeletal muscle α-actin promoter, cardiac α-actin promoter, myosin heavy chain (MyHC) promoter, myosin light chain 2 (MLC2) promoter, myosin light chain 3F promoter, desmin gene promoter, and myogenic regulatory factor family (MyoG, Myf5, Mrf4 and Myogenin); and osteoblast lineage-specific promoters (e.g. osteocalcin promoter).

As used herein, the term "inverted terminal repeat (ITR)" includes any AAV viral terminal repeat or synthetic sequence that forms a hairpin structure and functions as a cis-element to mediate viral replication, packaging, and integration. ITRs herein includes, but not limited to, the terminal repeats from AAV type 1-12 (terminal repeats from avian AAV, bovine AAV, canine AAV, equine AAV, and ovine AAV). In addition, the AAV terminal repeats do not have to have the native terminal repeat sequence, as long as the terminal repeat is functional for viral replication, packaging and integration.

As used herein, the term "cis-element" refers to a transgenic cassette packaged in AAV particles and expressed in target cells to produce a protein product with a therapeutic effect.

As shown in FIG. 1A, the full-length human dystrophin has four structural domains: the N-terminal domain, the central rod domain, which contains 24 spectrin-like repeats (R1-R24) and 4 hinges (H1-H4), a CR domain and the C-terminal (CT) domain. The full-length human dystrophin-associated protein also has four domains: the N-terminal domain, the central rod domain, which contains 22 spectrin-like repeats (R1-R22) and 4 hinges (H1-H4), a CR domain and the C-terminal (CT) domain. Their specific amino acid sequences are known in the art, and can be found in the literature or in public databases, such as the UniProt protein database.

The M6 construct of the present disclosure includes two forms: a M6 construct without codon optimization and a codon-optimized M6 (opti-M6) construct.

As used herein, the term "codon optimization" or "codon-optimized" refers to a polynucleotide sequence that has been modified from its native form. Such modifications result in one or more base pair differences, with or without changes in the corresponding amino acid sequence, that may enhance or inhibit gene expression and/or cellular response to the modified polynucleotide sequence.

In one embodiment, the AAV capsid protein can be an AAV capsid protein of any serotype, including native AAV capsid proteins (e.g., capsid proteins of native AAV type 1-11, avian AAV, bovine AAV, canine AAV, equine AAV, and ovine AAV) and engineered AAV capsid proteins (e.g., engineered capsid proteins of AAV type 1-11, avian AAV, bovine AAV, canine AAV, equine AAV and ovine AAV). Genome sequences, ITR sequences, Rep and Cap proteins of different AAV serotypes are known in the art. These sequences can be found in the literature or in public databases, such as the GenBank database and/or WO 2021050970 A1; US 2019/036676 A1; Choudhury SR et al., In Vivo Selection Yields AAV-B1 Capsid for Central Nervous System and Muscle Gene Therapy. Mol Ther., 2016, 24(7): 1247-57; Hsu HL. et al., Structural characterization of a novel human adeno-associated virus capsid with neurotropic properties. Nat Commun 11, 3279 (2020).

In one embodiment, the present disclosure provides a therapeutic tool for improving muscle functions, which can be used to treat a variety of diseases with relevant pathological mechanisms, including but not limited to: Duchenne muscular dystrophy, Baker muscular dystrophy, and other muscle degenerative diseases.

In one embodiment, clinical manifestations of muscle degenerative diseases include muscle atrophy and/or reduced or loss of exercise capacity.

In one embodiment, the protein products of the therapeutic tool (e.g., a transgenic expression cassette) include proteins for improving muscle functions, such as, but not limited to, (full-length or truncated) Dystrophin, (full-length or truncated) Utrophin and hybrid combinations of the two.

In one embodiment, the therapeutic tool is a protein, such as but not limited to, (full length or truncated) Dystrophin, (full length or truncated) Utrophin, and recombinant hybrid proteins of the two.

In one embodiment, the transgenic expression cassette of the present disclosure includes: a hCK promoter sequence (SEQ ID NO: 1), a mini polyadenylation (polyA) sequence (SEQ ID NO: 2) and a codon-optimized M6 (opti-M6) construct (SEQ ID NO:8), so as to form the hCK-opti-M6 expression cassette (SEQ ID NO:3). The hCK-opti-M6 expression cassette is flanked by a normal ITR of 145 bp, which enables the expression cassette to be packaged into AAV particles as a single-stranded AAV vector.

In one embodiment, the transgenic expression cassette of the present disclosure includes: a hCK promoter sequence (SEQ ID NO: 1), a mini polyadenylation (polyA) sequence (SEQ ID NO: 2) and a M6 construct without codon optimization (SEQ ID NO: 10), so as to form the hCK-M6 expression cassette (SEQ ID NO: 5). The hCK-M6 expression cassette is flanked by a normal ITR of 145 bp, which enables the expression cassette to be packaged into AAV particles as a single-stranded AAV vector.

In one embodiment, the transgenic expression cassette of the present disclosure includes: a shCK promoter sequence (SEQ ID NO: 9), a mini polyadenylation (polyA) sequence (SEQ ID NO: 2) and a codon-optimized M6 (opti -M6) construct (SEQ ID NO:8), so as to form the shCK-opti-M6 expression cassette (SEQ ID NO:6). The shCK-opti-M6 expression cassette is flanked by a normal ITR of 145 bp, which enables the expression cassette to be packaged into AAV particles as a single-stranded AAV vector.

In one embodiment, the transgenic expression cassette of the present disclosure includes: a shCK promoter sequence (SEQ ID NO: 9), a mini polyadenylation (polyA) sequence (SEQ ID NO: 2) and a M6 construct without codon optimization (SEQ ID NO: 10), so as to form the shCK-M6 expression cassette (SEQ ID NO: 7). The shCK-M6 expression cassette is flanked by a 145 bp normal ITR, which enables the expression cassette to be packaged into AAV particles as a single-stranded AAV vector.

In some embodiments, AAV particles of dystrophin and dystrophin-associated protein are produced by three-plasmid (Plasmid 1: cis-element plasmid; Plasmid 2: AAV Rep/Cap plasmid; Plasmid 3: helper plasmid) transfection of HEK293 cells.

In one embodiment, to produce therapeutic AAV particles, three-plasmid transfection of HEK293 cells is performed as follows: Plasmid 1: cis-element plasmid with ITR (e.g., hCK-opti-M6, hCK-M6, shCK-opti-M6 and shCK-M6 expression cassettes); Plasmid 2: AAV Rep/Cap plasmid with an encoding sequence for a capsid protein (e.g., AAV9 capsid protein); Plasmid 3: Helper plasmid with adenoviral components that can promote the replication, assembly, and packaging of AAV virions. In one embodiment, AAV particles produced by HEK293 cells are purified by cesium chloride (CsCl) density gradient centrifugation (e.g., the method as described in Example 2 of this disclosure).

Those skilled in the art can use known standard methods to produce recombinant and synthetic polypeptides or proteins, design nucleic acid sequences, produce transformed cells, construct recombinant AAV mutants, modify capsid proteins, and package vector expressing the AAV Rep and/or Cap sequences, as well as transiently or stably transfect packaging cells. These techniques are known to those skilled in the art. See, e.g., MOLECULAR CLONING: A LABORATORY MANUAL, 2nd ed. (Cold Spring Harbor, NY, 1989).

In some embodiments, the gene delivery systems of the present disclosure are used in adjunctive cell transplantation therapy. Specifically, AAV particles with transgenes can be used to transduce various types of cells *in vitro* to produce stable cell lines expressing protein products, which can then be introduced into the body for therapeutic purposes. The types of cells include, but are not limited to, endothelial cells, myoblasts, fibroblasts, astrocytes, Müller cells, oligodendrocytes, microglia, rods and cones, neurons, hematopoietic stem cells, monocytes, granulocytes, lymphocytes, osteoclasts and macrophages.

In one embodiment, the cells used for transplantation are autologous cells of the subjects, which allow for *in vitro* culture. The principles and techniques for introducing or transplanting cells into a subject are known to those skilled in the art.

In one embodiment, AAV particles are harvested from the culture medium and lysates of HEK293 cells. Purification methods include affinity chromatography, ion exchange chromatography, cesium chloride and iodixanol gradient ultracentrifugation. Chemicals or reagents associated with AAV production and purification include, but are not limited to: the chemicals or reagents used for cell culture (e.g., components of cell culture media including serum of bovine, equine, goat, chicken, or other vertebrate; glutamine, glucose, sucrose, sodium pyruvate, phenol red; antibiotic such as penicillin, kanamycin, streptomycin, tetracycline); the chemicals or reagents used for cell lysis, polynucleotide precipitation, or ultracentrifugation (e.g., Triton X-100, NP-40, sodium deoxycholate, sodium lauryl sulfate, domiphen bromide, sodium dodecyl salicylate, sodium chloride, magnesium chloride, calcium chloride, barium chloride, nitrates, potassium chloride, ammonium chloride, ammonium persulfate, ammonium sulfate, PEG-20, PEG-40, PEG-400, PEG-2000, PEG-6000, PEG-8000, PEG-20000, Tris-HCl, Tris-acetate, manganese chloride, phosphates, bicarbonates, cesium chloride, methanol, ethanol, glycerin, iodixanol, isopropyl alcohol, butanol, benzonase, DNase I, RNase); materials of affinity column (e.g. AAVX affinity resins, heparan sulfate proteoglycans and mucin resins, other materials associated with AAV-specific antibodies); acids, bases and organics contained in ion exchange chromatography materials and wash buffers (e.g. hydrochloric acid, sulfuric acid, acetic acid, formic acid, nitric acid, urea, acetone, chloroform, acetonitrile, trifluoroacetic acid, sodium hydroxide, potassium hydroxide, barium hydroxide, ammonium hydroxide, Tris base or other organic amines, poloxamer 188 , Tween 20, Tween 40, Tween 80, guanidine hydrochloride).

In one embodiment, the exogenous polynucleotide delivered to the target cell by the AAV vector encodes a native protein for therapeutic use, which may or may not be codon optimized.

In one embodiment, the exogenous polynucleotide delivered to the target cell by the AAV vector encodes a synthetic polypeptide.

In one embodiment, the transgenic expression cassette or gene delivery system of the present disclosure is formulated into pharmaceutical compositions (e.g., injections, tablets, capsules, powders, eye drops) for administration to humans or other mammals. The pharmaceutical compositions also contain other ingredients, such as pharmaceutical excipients, aqueous or organic solvents (such as water, glycerol, ethanol, methanol, isopropyl alcohol, chloroform, phenol or polyethylene glycol), salts (such as sodium chloride, potassium chloride, phosphates, acetates, bicarbonates, Tris-HCl and Tris-acetate), reagents for retarding dissolution (e.g. paraffin), surfactants, antimicrobials, liposomes, lipoplexes, immune inhibitors (e.g., cortisone, prednisone, cyclosporine), nonsteroidal anti-inflammatory medicaments (NSAIDs, e.g., aspirin, ibuprofen, acetaminophen) microspheres, rigid matrices, semi-solid carriers, nanospheres or nanoparticles. In addition, the pharmaceutical composition can be delivered at a single dose or multiple doses through inhalation, systemic or local (e.g., intravenous, subcutaneous, intraocular, intravitreal, subretinal, suprachoroidal, parenteral, intramuscular, intracerebroventricular, oral, intraperitoneal, and intrathecal) administration.

In one embodiment, the present disclosure provides a pharmaceutical composition, comprising the recombinant hybrid protein, nucleic acid molecule, transgenic expression cassette or gene delivery system of the present disclosure; and excipient(s). The pharmaceutical composition of the present disclosure can be used to transduce cells *in vitro* or transduce mammals (such as rodents, primates, and humans) *in vivo* to treat various diseases relevant to muscle deficits, such as muscular dystrophies, including: Duchenne muscular dystrophy, Becker muscular dystrophy, and other muscle degenerative diseases. In one embodiment, the muscular dystrophy is Duchenne muscular dystrophy.

In one embodiment, the treatment of muscular dystrophy refers to the improvements in the degree of muscle damage, creatine kinase levels, and NASS (The North American Spine Society) muscle function score in the patients receiving the treatment.

The present disclosure will be described in further detail below with reference to the accompanying figures and examples. The following examples are only used to illustrate the present disclosure and are not intended to limit the scope of the present disclosure. Experimental methods without specifying specific conditions in the examples were performed in accordance with conventional conditions known in the art, or in accordance with conditions recommended by the manufacturer.

### Examples

### Example 1: Design and Construction of M6 and opti-M6 Constructs, and AAV Vectors

The B84 (Mini-dystrophin) construct (SEQ ID NO: 11) was obtained by PCR cloning method in use of human dystrophin cDNA as a template. The B84 construct and the mini-human dystrophin protein encoded by the B84 conduct are described in e.g., CN 109641944A (in the name of Hopti-Dys3978 and Dys3978, respectively) or Wang B, Li J, Xiao X. Adeno-associated virus vector carrying human minidystrophin genes effectively ameliorates muscular dystrophy in mdx mouse model[J]. Proc Natl Acad Sci USA, 2000, 97(25): 13714-9.

Through extensive experimental researches and rational designs, the inventors also constructed a highly truncated M6 construct (SEQ ID NO: 10) based on the full-length human dystrophin and the full-length human dystrophin-associated protein, and obtained an opti-M6 construct (SEQ ID NO: 8) through codon optimization on the M6 construct. As shown in FIG. 1A, the recombinant hybrid protein encoded by the M6 construct or the opti-M6 construct comprises: the N-terminal domain, hinge H1, spectrin-like repeat R1, spectrin-like repeat R2, spectrin-like repeat R3 and the first half of hinge H2 of the full-length human dystrophin-associated protein (Utrophin); and the spectrin-like repeat R23, spectrin-like repeat R24, hinge H4, and CR domain of the full-length human Dystrophin.

In addition, in order to enhance the expression of the target protein, the inventors further constructed a shortened human creatine kinase CK (shCK) promoter based on the human creatine kinase hCK promoter (SEQ ID NO: 1).

Then, the above-mentioned M6 and opti-M6 genes were subcloned into AAV vector plasmids, in use of the hCK promoter and shCK promoter respectively, to generate AAV-hCK-M6-polyA, AAV-hCK-opti-M6-polyA, AAV-shCK-M6-polyA and AAV-shCK-opti-M6-polyA vectors. Similarly, the B84 gene was cloned into an AAV vector plasmid containing the hCK promoter and mini polyA signal sequence, to generate AAV-hCK-B84 vector.

### Example 2: Expression of M6 and opti-M6 Constructs in Mdx Mice

In order to study the expression of M6 and opti-M6 constructs in mice, four treatment groups were set in this example: hCK-opti-M6, hCK-M6, shCK-opti-M6 and shCK-M6.

The AAV-hCK-M6-polyA, AAV-hCK-opti-M6-polyA, AAV-shCK-M6-polyA and AAV-shCK-opti-M6-polyA vectors constructed in Example 1 were packaged into AAV9 virus particles. AAV virus were produced according to the three-plasmid cotransfection method (see e.g., Xiao X, Li J, Samulski R J. Production of high-titer recombinant adeno-associated virus vectors in the absence of helper adenovirus[J]. J Virol, 1998, 72(3): 2224-32) by three-plasmid transfection of the above-mentioned four new cis-element plasmid vectors, AAV9 capsid plasmid and adenovirus helper plasmid. The HEK293 cells were lysed after 40-80 hours' transfection, and the polynucleotides were removed. The AAV viral vectors were subsequently purified twice by CsCl density gradient ultracentrifugation according to the protocol described in Snyder R, Xiao S, Samulski R J. Production of recombinant adeno-associated viral vectors[J], 1996. The virus titer was measured by qPCR method and was approximately 2×10¹³~1×10¹⁴ vg/ml. Capsid protein purity was determined by SDS-PAGE. The presence of endotoxin was determined by gel method, and the results were qualified.

Then, the above-mentioned AAV particles were injected at 3×10¹³vg/kg into the Mdx mice through the tail vein. WT mice and littermate Mdx mice of the same age were used as controls. Six weeks after injection, mice were dissected. Tissues of heart, gastrocnemius, biceps brachii, and diaphragm were collected for frozen sectioning and immunofluorescence staining. The rabbit-derived polyclonal antibody used for immunofluorescence staining was purchased from Abcam (catalog number ab251754), which can recognize the amino acid fragment at positions 2800-3000 of human dystrophin (spectrin-like repeat R23 and R24). Therefore, the proteins expressed by both B84 and M6 constructs can be recognized by this antibody.

Immunofluorescence staining of tissues of heart, gastrocnemius, biceps brachii and diaphragm can respectively reflect the improvements in muscle pathology relevant to cardiac function, lower limb motor function, upper limb motor function and respiratory function of Mdx mice. The pathological phenotype of Mdx mice began at about 3 weeks of age, accompanied by the degeneration and regeneration of muscle fibers. The characteristic manifestation and main pathological manifestation of this process is that the nucleus is located in the center of the muscle fibers.

The results show that, in the hearts of Mdx mice after six weeks of treatment, the protein expression levels in the four treatment groups were significantly higher than those in the untreated Mdx control group. Among them, the expression levels of the hCK-opti-M6 and shCK-opti-M6 expression cassettes were higher than that of the hCK-M6 and shCK-M6 expression cassettes (FIG. 2A). Similarly, the expression of hybrid therapeutic proteins was also observed in the gastrocnemius muscle of mice after treatment, and the expression level of opti-M6 was significantly higher than that of un-optimized M6 (FIG. 2B). In biceps brachii, the protein expression level in each of the four treatment groups was significantly higher than that in the untreated Mdx control groups. shCK-opti-M6 was significantly better than the other three treatment groups, with an expression level close to that in WT mice, and significantly corrected the centronucleation phenomenon in skeletal muscle of Mdx mice (FIG. 2C). The expression in diaphragm was similar to that in biceps brachii, shCK-opti-M6 was widely expressed in the visual field and was better than the other three expression cassettes (FIG. 2D).

The above results show that M6 and opti-M6 constructs can widely express hybrid therapeutic proteins in four representative muscle tissues, including heart and gastrocnemius, and produce therapeutic effects. In comparison with the M6 construct, the expression level of the optimized opti-M6 construct was higher. Moreover, in comparison with the hCK, the use of shCK can obtain a higher protein expression level. In summary, the shCK-opti-M6 expression cassette has the best *in vivo* activity.

### Example 3: Expression of Opti-M6 Construct and B84 Construct in Mdx mice

In this example, the inventors compared the expression levels of the opti-M6 construct and the B84 construct in model mice. Opti-M6 and B84 were separately constructed into the vector having hCK promoter, and packaged into viruses to obtain AAV9-hCK-opti-M6 and AAV9-hCK-B84 virus particles. Mdx mice were treated with virus particles via tail vein injection at a dose of 3×10¹³ vg/kg. WT mice and littermate Mdx mice of the same age were used as controls. After 6 weeks of treatment, the mice were dissected, and tissues of heart, gastrocnemius muscle, diaphragm, and etc. were collected. The expressions of therapeutic proteins in different tissues were detected by tissue immunofluorescence.

The results show that the expression levels of therapeutic proteins in the heart, gastrocnemius and diaphragm of mice treated with AAV9-hCK-opti-M6 and AAV9-hCK-B84 were significantly higher than those in the untreated mdx control group (FIG. 3).

As shown in FIG. 3, in heart, the AAV9-hCK-opti-M6 treatment group achieved a fluorescence intensity similar to the AAV9-hCK-B84 treatment group, and had more positive cell; in the gastrocnemius muscle, the fluorescence intensity of the AAV9-hCK-opti-M6 treatment group was significantly stronger than that of the AAV9-hCK-B84 treatment group; in the diaphragm, compared with the AAV9-hCK-B84 treatment group, the AAV9-hCK-opti-M6 treatment group had stronger fluorescence intensity and significantly higher number of positive cells.

In summary, the opti-M6 construct produced better therapeutic effects compared with the B84 construct.

### Example 4: Expression of shCK-opti-M6 Vector and hCK-B84 Vector in Mdx Mice

In this example, the inventors compared the expression capabilities of the shCK-opti-M6 vector and the hCK-B84 vector in the model mice.

As described in Example 2, AAV-shCK-opti-M6 and AAV-hCK-B84 vectors were separately packaged into the AAV9-shCK-opti-M6 and the AAV9-hCK-B84 virus particles. The Mdx mice were treated with the virus particles via tail vein injection at a dose of 3×10¹³ vg/kg. WT mice and littermate Mdx mice of the same age were used as controls. After 4 weeks of treatment, the mice were dissected, and tissues of heart, gastrocnemius, quadriceps, diaphragm, and etc. were collected. Then, immunofluorescence and Western blotting were used to determine protein expression in different tissues.

As shown in FIG. 4A, the expression levels of therapeutic proteins in the hearts of mice treated with AAV9-shCK-opti-M6 and AAV9-hCK-B84 were significantly higher than those in the untreated Mdx control groups, with AAV9-shCK-opti-M6 having stronger fluorescence.

As shown in FIGS. 4B to 4E, widespread expressions of therapeutic proteins was also observed in limb musculature (gastrocnemius, tibialis anterior, biceps brachii, and quadriceps) after treatments with AAV9-shCK-opti-M6 and AAV9-hCK-B84. The expression level of the shCK-opti-M6 treatment group in limb muscle tissue was significantly higher than that of the hCK-B84 treatment group. In mice in the shCK-opti-M6 treatment group, the inventors observed peripheral nucleation of muscle fibers in areas where the proteins were widely expressed, indicating that the pathological phenotype of centronucleation was corrected to some extent, and muscle degeneration and regeneration were prevented, and indicating that treatment with the vector can improve the pathological phenotypes of muscles.

As shown in FIGS. 4F to 4H, in the diaphragm and intercostal muscle tissues relevant to respiratory function, widespread expression of therapeutic proteins was observed in both shCK-opti-M6 and hCK-B84 treatment groups, with no significant difference in fluorescence intensity between the two.

The protein expression of shCK-opti-M6 and hCK-B84 *in vivo* was simultaneously detected using antibodies that are capable of recognizing the spectrin-like repeat R23 and R24 fragments of human dystrophin. Western blot results show that the expression level of shCK-opti-M6 was higher than that of hCK-B84 in the heart of mice, and the expression level of shCK-opti-M6 in the gastrocnemius muscle and diaphragm were close to that of hCK-B84 (FIG. 4G).

Taken together, the above results show that, shCK-opti-M6 has a significant advantage in expression compared with hCK-B84,.

### Example 5: Creatine Kinase Level in Mdx Mice Treated with shCK-opti-M6 Vector and hCK-B84 Vector

In this example, in order to verify the therapeutic effects of the shCK-opti-M6 and hCK-B84 vectors in Mdx mice, the inventors detected the serum creatine kinase (CK) level, which is an important indicator of muscle damage in Mdx mice.

Two treatment groups were set in this example: AAV9-shCK-opti-M6 and AAV9-hCK-B84. Mdx mice were treated via tail vein injection at a dose of 3×10¹³ vg/kg. WT mice and littermate Mdx mice of the same age were used as controls. There are 8 animals in each group: 4 males and 4 females. After 8-9 weeks of treatment, serum was collected through orbital blood collection to detect the CK level in the serum of mice.

As shown in FIG. 5, the serum CK level of untreated Mdx mice was significantly higher than that of WT mice at 4 months of age (male, **p<0.01; female, ***p<0.001), while the serum CK level was significantly reduced in Mdx mice treated with AAV, with the serum CK value of shCK-opti-M6 treatment group significantly reduced (male, *p<0.05; female, ***p<0.001).

It can be seen that the creatine kinase level of Mdx mice treated with shCK-opti-M6 vector was lower than that of Mdx mice treated with hCK-B84 vector, indicating that shCK-opti-M6 vector has a better therapeutic effect.

### Example 6: Effects of shCK-opti-M6 and hCK-B84 Vectors on Exercise Capacity and Muscle Strength of Mdx Mice

In this example, in order to evaluate the effects of shCK-opti-M6 and hCK-B84 vectors on the exercise capacity and muscle strength of Mdx mice, the mice were subjected to the rotarod test (movement) and the grip test (strength).

Three repeat rotarod tests were performed in WT mice, untreated Mdx mice, and Mdx mice after 8 weeks of treatment with shCK-opti-M6 and hCK-B84 (n=4). The results are shown in FIG. 6A, there is a significant difference in the duration on the rotarod between untreated Mdx mice and WT mice (*p<0.05). Compared with the untreated Mdx mice, the movement performance of the shCK-opti-M6 vector-treated Mdx mice was significantly improved (*p<0.05), and the hCK-B84 vector-treated Mdx mice has no statistical difference. Notably, the shCK-opti-M6 vector-treated Mdx mice shows a duration of movement close to that of the WT mice.

Meanwhile, the results of the mouse grip force test show that, the grip force of the limbs of Mdx mice treated with shCK-opti-M6 vector was significantly improved compared with untreated Mdx mice (**p<0.01) and was close to the level of WT mice (FIG. 6B).

The above results indicate that, shCK-opti-M6 vector is more effective in improving the muscle functions and behavioral performances of mice compared with hCK-B84 vector.

### Example 7: Long-term Expression of shCK-opti-M6 Vector in Mdx Mice

In this example, the inventors studied the long-term expression of the shCK-opti-M6 vector in Mdx mice. Four groups of Mdx mice were administered by tail vein injection at a dose of 3×10¹³ vg/kg. Muscle samples were collected at different time points (4 weeks, 6 weeks, 9 weeks, and 12 weeks) after the injection of the vector for immunofluorescence and Western blot detection. WT mice and littermate Mdx mice of the same age were used as controls.

The results show that protein expression could be observed in the heart after 4 weeks of treatment with shCK-opti-M6 vector. Expression reached a peak period at 6-9 weeks after injection, and the number of positive cells and fluorescence intensity increased significantly (FIG. 7A). Widespread protein expression was observed in the gastrocnemius muscle of mdx mice after 6 weeks of shCK-opti-M6 vector treatment (FIG. 7B). After 4 weeks of shCK-opti-M6 vector treatment, weak fluorescence intensity was observed in the quadriceps muscle, and fluorescence expression reached a peak period at 6-9 weeks after injection and continued stably until 12 weeks after injection (FIG. 7C). Biceps brachii and quadriceps have similar immunofluorescence staining results (FIG. 7D). In the tibialis anterior muscle of Mdx mice, high levels of protein expression were already shown at 4 weeks post-injection, and expression levels further increased at 6-9 weeks post-injection, and last till 12 weeks post-injection (FIG. 7E). Diaphragm and tibialis anterior muscle have similar immunofluorescence staining results (FIG. 7F). Immunofluorescence staining results of intercostal muscles show that, high-level protein expression was detected from 4 to 9 weeks after shCK-opti-M6 vector treatment, and the expression decreased slightly at 12 weeks after injection (FIG. 7G). Tongue and intercostal muscles have similar immunofluorescence results (FIG. 7H).

Antibodies that recognize the spectrin-like repeat R23 and R24 fragments of human dystrophin were used to detect protein expression, and the results of Western blot analysis were shown in FIG. 7I. In heart, protein expression can be detected as early as 4 weeks post-injection, with the highest expression levels at 6-9 weeks and last until 12 weeks post-injection. In gastrocnemius muscle, there was high expression level at 6 weeks post-injection, and the expression reached its peak at 12 weeks post-injection. In quadriceps muscle, protein expression reached its peak period at 9 weeks post-injection, and maintained the high expression level at 12 weeks post-injection. In biceps brachii, protein expression levels were highest at 6 weeks post-injection, and relatively high levels of expression could still be detected at 12 weeks post-injection. In tibialis anterior muscle, a certain level of protein expression can be detected at 4 weeks post-injection, and the protein expression maintained at a high level during 6-9 weeks post-injection. In diaphragm, protein expression was still detectable at 12 weeks post-injection. The results of Western blot analysis of the intercostal muscles were similar to those of the tongue, that are, different levels of protein expression were detected at 4-9 weeks post-injection, and the expression levels decreased at 12 weeks post-injection.

The above immunofluorescence and Western blot results show that shCK-opti-M6 can achieve a long-term stable expression in the Mdx model mice.

Although the present disclosure has been illustrated and described with reference to the accompanying figures and preferred embodiments of the present disclosure, those of ordinary skill in the art should understand that, the above content is a further detailed description of the present disclosure in conjunction with specific embodiments, and the invention should not be constructed as limited to the embodiments set forth herein. Those skilled in the art may make various changes in both form and detail, including making several simple deductions or substitutions, without departing from the spirit and scope of the present disclosure.

## Claims

1. A recombinant hybrid protein, comprising:
N-terminal domain, hinge H1, spectrin-like repeat R1, spectrin-like repeat R2, spectrin-like repeat R3 and a first half of hinge H2 of a full-length human Utrophin; and
spectrin-like repeat R23, spectrin-like repeat R24, hinge H4, and CR domain of a full-length human Dystrophin.

2. The recombinant hybrid protein of claim 1, wherein the recombinant hybrid protein comprises an amino acid sequence as shown in SEQ ID NO: 4; preferably the recombinant hybrid protein consists of the amino acid sequence as shown in SEQ ID NO: 4.

3. A nucleic acid molecule encoding the recombinant hybrid protein of claim 1 or 2.

4. The nucleic acid molecule of claim 3, wherein the nucleic acid molecule has a nucleotide sequence at least 50% identical to the nucleotide sequence of SEQ ID NO: 8 or SEQ ID NO: 10, preferably at least 50%, 60%, 70%, 80%, 85%, 90%, 95%, 99% or 100% identical to the nucleotide sequence shown in SEQ ID NO: 8 or SEQ ID NO: 10.

5. The nucleic acid molecule of claim 4, wherein the nucleic acid molecule comprises the nucleotide sequence of SEQ ID NO: 8 or SEQ ID NO: 10, preferably the nucleic acid molecule consists of the nucleotide sequence as shown in SEQ ID NO: 8 or SEQ ID NO: 10.

6. A transgenic expression cassette comprising promoter(s), the nucleic acid molecule of any one of claims 3 to 5, and a mini polyA.

7. The transgenic expression cassette of claim 6, wherein the promoter is selected from: CB promoter, CAG promoter, promoters of muscle-specific genes including muscle creatine kinase (MCK) promoter, human creatine kinase (hCK) promoter, shortened human creatine kinase (shCK) promoter, skeletal muscle α-actin promoter, cardiac α-actin promoter, myosin heavy chain (MyHC) promoter, myosin light chain 2 (MLC2) promoter, myosin light chain 3F promoter, desmin gene promoter, and myogenic regulatory factor family (MyoG, Myf5, Mrf4 and Myogenin); preferably, the promoter is hCK promoter or shCK promoter; more preferably, the promoter is shCK promoter.

8. The transgenic expression cassette of claim 6 or 7, wherein the promoter has a nucleotide sequence as shown in SEQ ID NO: 9.

9. The transgenic expression cassette of any one of claims 6 to 8, wherein the transgenic expression cassette further comprises regulatory elements, such as two ITRs located at its both ends, each of which is either a normal ITR or a shortened ITR; preferably both of which are normal ITRs of 145 bp.

10. The transgenic expression cassette of any one of claims 6 to 9, wherein the transgenic expression cassette further comprises an origin of replication, a polyadenylation signal, an internal ribosome entry site (IRES), and/or a 2A signal, such as P2A, T2A and F2A.

11. The transgenic expression cassette of any one of claims 6 to 10, wherein the transgenic expression cassette consists of the nucleotide sequence as shown in SEQ ID NO: 3, SEQ ID NO: 5, SEQ ID NO: 6 or SEQ ID NO: 7.

12. A gene delivery system comprising the transgenic expression cassette of any one of claims 6 to 11 and an AAV capsid protein.

13. The gene delivery system of claim 12, wherein the AAV capsid protein is a natural AAV capsid protein or an engineered AAV capsid protein; preferably, the AAV is selected from: AAV1, AAV2, AAV3, AAV4, AAV5, AAV6, AAV7, AAV8, AAV9, AAV10, AAV11, AAV12, AAV-DJ, AAV-DJ8, AAV-DJ9, AAVrh8, AAVrh8R, AAVrh10, AAVrh39, AAVrh43, AAV32.33, AAV3B, AAVv66, AAVXL32 and AAV.PHP.B.

14. Use of the transgenic expression cassette of any one of claims 6 to 11, or the gene delivery system of claim 12 or 13 in the preparation of a pharmaceutical composition for treating muscular dystrophy.

15. The use of claim 14, wherein the muscular dystrophy comprises: Duchenne muscular dystrophy, Becker muscular dystrophy, and other muscle degenerative diseases; preferably, the muscular dystrophy is Duchenne muscular dystrophy.

16. A pharmaceutical composition comprising one of the recombinant hybrid protein of claim 1 or 2, the nucleic acid molecule of any one of claims 3 to 5, the transgenic expression cassette of any one of claims 6 to 11, and the gene delivery system of claim 12 or 13; and excipient(s).

17. A method of treating muscular dystrophy, comprising administering to a subject in need thereof a therapeutically effective amount of the pharmaceutical composition of claim 16.

18. The method of claim 17, wherein the pharmaceutical composition is administered through systemic or local routes, such as by intravenous administration, intramuscular administration, subcutaneous administration, oral administration, topical administration, intraperitoneal administration or intralesional administration; preferably, the pharmaceutical composition is administered systemically, such as by intravenous administration; more preferably, the pharmaceutical composition is locally administered to muscles, such as by biceps brachii injection or gastrocnemius muscle injection.
